# EUROPEAN PATENT APPLICATION

(11) **EP 2 610 239 A1**
(43) Date of publication of application: **03.07.2013**
(21) Application number: 12008580.8
(22) Date of filing: 21.12.2012
(51) Int. Cl.: C07C 209/68, C07C 209/84, C07C 211/42

(54) **Preparation Of Rasagiline Hemitartrate**

(30) Priority: 02.01.2012 IN CH00122012
(71) Applicant: Dr. Reddy's Laboratories Ltd., 500 034 Andhra Pradesh (IN)
(72) Inventor: Gade, Srinivas Reddy, 500072 Hyderabad (IN); Adla, Vijay Kumar, 500017 Secunderabad (IN); Vempati, Chandrasekhar, 520003 Andhra Pradesh (IN)
(74) Representative: Müller-Boré & Partner Patentanwälte

(57) **Abstract**

The present application provides improved processes for crystallization of Rasagiline hemitartrate having improved filterability.

## Description

Aspects of the present invention relate to rasagiline hemitartrate, to rasagiline hemitartrate with improved filterability and with advantageously reduced fluffiness, to processes for the preparation thereof, and to the use thereof for milling and/or directly for preparing pharmaceutical formulations. Methods for improving the filterability and/or alleviating the stickiness of rasagiline hemitartrate having a very poor flowability and/or showing undesirable stickiness are also disclosed.

The drug compound having the adopted name "rasagiline" has a chemical name (1R)-N-prop-2-ynyl-2,3-dihydro-1H-inden-1-amine, and can be represented by structural Formula I.

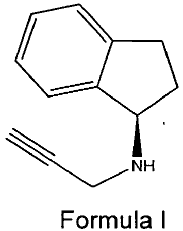

Rasagiline in the form of mesylate salt is prescribed for the treatment of idiopathic Parkinson's disease.

U.S. Patent Nos. 3,253,037, 5,457,133, and 5,532,415 pertain to racemic rasagiline, its enantiomer and salts like tartrate, esylate, sulfate and mesylate.

WO 2008/076348 A1 discloses crystalline rasagiline free base, a process for its preparation and a pharmaceutical composition comprising crystalline rasagiline free base.

WO 2010/059913A2 discloses preparation of Rasagiline hemitartrate and mesylate salts.

The solid state physical properties of an active pharmaceutical ingredient (API), such as rasagiline salt, can be very important in formulating a drug substance and can have profound effects on the ease of formulation. Particle size, for example, may affect the filterability and mixability of a drug substance. Additionally, pharmaceutical stability is believed to depend on simultaneous influence of various factors, of which some important factors include the sizes of crystals, shapes of crystals, water content, residual solvents, and impurities.

Additional parameter is the flowability that affects the ease with which a material is handled during processing into a pharmaceutical product. Namely, when flowability is very poor, problems may occur with handling and processing during the milling and formulating.

U.S. Patent Application Publication No. 2006/0188581 is directed to a mixture of particles of pharmaceutically acceptable salts of Rasagiline, wherein more than 90% of the total amount by volume of particles of Rasagiline salt have a size less than 250 µm. The application emphasizes the concern of maintaining uniformity of content during formulation of low dosage strength drugs like Rasagiline mesylate. It also highlights that the large, irregularly shaped particles arising from salt crystallization can easily decrease content uniformity in formulated products.

WO 2006/091657 is directed to a mixture of particles of pharmaceutically acceptable salts of Rasagiline, wherein more than 90% of the total amount by volume of particles of Rasagiline salt have a size less than 250 µm. The application emphasizes the concern of maintaining uniformity of content during formulation of low dosage strength drugs like Rasagiline mesylate. It also highlights that the large, irregularly shaped particles arising from salt crystallization can easily decrease content uniformity in formulated products. WO 2006/091657 thus teaches that mechanical comminution is the specific process which provides rasagiline having a D₉₀ of less than 250 microns, which leads to an improved content uniformity of the tablet.

Further, WO 2007/061717 describes preparation of rasagiline hemitartrate having rod shaped morphology by recrystallization from water. In particular, Examples 18 and 19 of this reference describes the preparation of rasagiline hemitartrate by prior art processes and its comparison with the rasagiline tartrate obtained in Example 15 by recrystallization of hemitartrate salt from water. Thus, Rasagiline hemitartrate as obtained by the processes disclosed in the above discussed prior art, which make use of methanol-isopropanol or methanol-methyl tert-butyl ether as a crystallization solvent, shows very poor filterability and to improve upon that WO2007/061717 disclosed an additional step of recrystallization from water to result in rod shaped morphology of particles to provide better processability of the product.

Filterability and flowability affects the ease with which a material is handled during processing into a pharmaceutical product. Namely, when flowability is very poor, problems may occur with handling and processing during the milling and formulating.

Thus, in view of the foregoing there is a need to provide rasagiline hemitartrate with improved filterability as well as flowability without an additional step of recrystallization and which also alleviates the problems associated with known rasagiline compositions on storage.

This object is solved by the embodiments defined in the claims.

Aspects of the present invention provide rasagiline hemitartrate, preferably crystals of rasagiline hemitartrate with improved filterability and processes for their preparation.

In particular, the present invention provides a process for preparation of rasagiline hemitartrate having improved filterability, comprising:
(a) providing a mixture of rasagiline in methanol;
(b) treating the mixture of step (a) with L-tartaric acid;
(c) combining the mixture of step (b) with methyl ethyl ketone and maintaining the mixture at a suitable temperature for a suitable time for crystallization;
(d) recovering the solid rasagiline tartrate from step (c).

The terms such as "about," "generally," "substantially," and the like are to be construed as modifying a term or value such that it is not an absolute. When a molecule or other material is identified herein as "substantially pure," it generally means, unless specified otherwise, that the material is at least about 95% pure, as determined by methods that are conventional in the art such as high performance liquid chromatography (HPLC) or spectroscopic methods. "Pure" generally means, unless specified otherwise, that the material is at least about 99% pure, as determined by such conventional methods. In general, this refers to purity with regard to unwanted residual solvents, reaction by-products, impurities, and unreacted starting materials.

Percentage values are used herein to denote percent by weight, unless the context indicates otherwise.

An aspect of the present invention provides processes for preparation of rasagiline hemitartrate having improved filterability, embodiments comprising at least one of the steps:
(a) providing a mixture of rasagiline in methanol;
(b) treating the mixture of step a) with L-tartaric acid;
(c) combining the mixture of step (b) with methyl ethyl ketone and maintaining the mixture at a suitable temperature for a suitable time for crystallization;
(d) recovering the solid rasagiline hemitartrate from step (c).

Step (a) involves providing a mixture of rasagiline in methanol.

The mixture of rasagiline in step (a) may be obtained by dissolving rasagiline in methanol. Alternatively, the mixture comprising rasagiline may be obtained directly from a reaction in which rasagiline is formed.

The mixture can be heated up to reflux temperature as long as the stability of rasagiline is not compromised and a clear solution is obtained. Time period can be as long as required for a complete dissolution.

The solution can optionally be filtered by passing through paper, glass fiber, or other membrane material, or a bed of a clarifying agent such as celite. Depending upon the equipment used and the concentration and temperature of the solution, the filtration apparatus may need to be heated to avoid premature crystallization.

Step (b) involves treating the mixture of step (a) with L-tartaric acid.

L-tartaric acid can be directly added as a solid or as a solution in suitable solvent, preferably methanol.

In a preferred embodiment, solution of L-tartaric acid is slowly added to a mixture of Rasagiline free base.

Step c) involves combining the solution of step b) with methyl ethyl ketone.

The rasagiline hemitartrate solution of step b) is combined with methyl ethyl ketone. The said step includes both mode of addition i.e. methyl ethyl ketone can be added to solution of step (b) or reverse mode of addition wherein a mixture of step b) is added to methyl ethyl ketone.

The mixture may first be heated to reflux temperature for a suitable time followed by cooling and stirring for solid formation. Suitable times for crystallization will vary and can be from about 10 minutes to about 1 hour, to about 24 hours, or longer. Suitable temperatures for crystallization include from about -10°C to about 35°C or from about 10°C to about 20°C. Alternately, stepwise cooling can be done to ease the filtration by improving the morphology of crystalline particles. The exact temperatures and time required for complete solid formation can be readily determined by a person skilled in the art.

Step d) involves recovering rasagiline hemitartrate from step c).

The methods by which the solid material is recovered from the final mixture, with or without cooling below the operating temperature, can be any of techniques such as decantation, filtration by gravity or suction, centrifugation, and the like. The isolated crystals may carry a small proportion of occluded mother liquor containing a higher percentage of impurities. If desired, the isolated crystals may be washed with a solvent to wash out the mother liquor.

Another aspect of the present invention provides processes for preparation of rasagiline hemitartrate, comprising :
(a) providing a mixture of rasagiline hemitartrate in methanol;
(b) combining the mixture of step (a) with methyl ethyl ketone and maintaining the mixture at a suitable temperature for a suitable time for crystallization;
(c) recovering the solid rasagiline hemitartrate from step (b).

The mixture of rasagiline hemitartrate in step (a) may be obtained by dissolving rasagiline hemitartrate in methanol. Alternatively, the mixture comprising rasagiline hemitartrate may be obtained directly from a reaction in which rasagiline hemitartrate is formed.

The wet cake obtained by above processes may optionally be further dried. Drying can be suitably carried out in a tray dryer, vacuum oven, air oven, fluidized bed dryer, spin flash dryer, flash dryer and the like. The drying can be carried out at temperatures of about 30°C to about 90°C, with or without applying vacuum. The drying can be carried out for any desired times until the desired product purity is achieved.

The isolated crystalline rasagiline hemitartrate can be present in any form which include but not limited to the anhydrate, a solvate, or a hydrate.

The Rasagiline hemitartrate of the present invention have potential to serve as intermediates in the purification of free base or preparation of other pharmaceutically acceptable acid addition salts.

The rasagiline salt is substantially free from impurities. Typically, the rasagiline salt is of high purity, such as at least about 99%, 99.5%, or 99.9%, by weight pure. Correspondingly, the level of impurities may be less than about 1 %, 0.5%, or 0.1 %, by weight, as determined using high performance liquid chromatography (HPLC).

In yet another aspect, rasagiline hemitartrate, preferably crystals of rasagiline hemitartrate with improved filterability and aspect ratio of more than 15 characteristics are disclosed.

The inventors have also surprisingly found that the rasagiline hemitartrate obtained by the processes disclosed herein shows a reduced specific surface area than the rasagiline hemitartrate obtained by the prior art processes which employ crystallization from methanol-isopropanol or methanol-MTBE.

In another aspect, the use of rasagiline hemitartrate with improved filterability and reduced fluffiness as a starting material for preparing a composition comprising rasagiline hemitartrate is disclosed.

In another further aspect, the present disclosure relates to a method for improving the filterability of rasagiline hemitartrate crystals with non-acceptable very poor flowing character comprising crystallizing the rasagiline hemitartrate by employing aforementioned solvent system viz., methanol and methyl ethyl ketone according to the process of the present as described above.

The present invention includes rasagiline hemitartrate formulated as: solid oral dosage forms, such as, for example, powders, granules, pellets, tablets, capsules; liquid oral dosage forms, such as, for example, syrups, suspensions, dispersions, emulsions; and injectable preparations, such as, for example, solutions, dispersions, freeze dried compositions Immediate release compositions may be conventional, dispersible, chewable, mouth dissolving, or flash melt preparations. Modified release compositions may comprise hydrophilic and/or hydrophobic release rate controlling substances to form matrix and/or reservoir systems. The compositions may be prepared using techniques such as direct blending, dry granulation, wet granulation, or by extrusion and spheronization. Compositions may be either uncoated, film coated, sugar coated, powder coated, enteric coated, or modified release coated.

Pharmaceutical compositions of rasagiline comprise one or more pharmaceutically acceptable excipients. Pharmaceutically acceptable excipients include, but are not limited to: diluents, such as, for example starches, pregelatinized starches, lactose, powdered celluloses, microcrystalline celluloses, dicalcium phosphate, tricalcium phosphate, mannitol, sorbitol, sugar, and the like; binders, such as, for example acacia, guar gum, tragacanth, gelatin, polyvinylpyrrolidones, hydroxypropyl celluloses, hydroxypropyl methylcelluloses, pregelatinized starches, and the like; disintegrants, such as, for example starches, sodium starch glycolate, pregelatinized starches, crospovidones, croscarmellose sodiums, colloidal silicon dioxides, and the like; lubricants, such as, for example stearic acid, magnesium stearate, zinc stearate, and the like; glidants, such as, for example colloidal silicon dioxides, and the like; solubility or wetting enhancers, such as, for example anionic, cationic, and neutral surfactants; complex forming agents, such as, for example various grades of cyclodextrins; release rate controlling agents, such as, for example hydroxypropyl celluloses, hydroxymethyl celluloses, hydroxypropyl methylcelluloses, ethyl celluloses, methyl celluloses, various grades of methyl methacrylates, waxes, and the like. Other pharmaceutically acceptable excipients include, but not limited to, film formers, plasticizers, colorants, flavoring agents, sweeteners, viscosity enhancers, preservatives, antioxidants, and the like.

Certain specific aspects and embodiments of the present invention will be explained in more detail with reference to the following examples, which are provided solely for purposes of illustration and are not to be construed as limiting the scope of the invention in any manner.

### EXAMPLES

### EXAMPLE 1: PREPARATION OF RASAGILINE HEMITARTRATE.

Rasagiline free base (5.0 g) and methanol (75 mL) were charged in a flask followed by addition of L-(+)-tartaric acid (1.95 g) and mixture was stirred for 10 minutes. To the mixture, methyl ethyl ketone (25 mL) was added and mixture was heated to reflux temperature followed by stirring at the same for 1 hour. The reaction mixture was cooled to room temperature and maintained at the same for 1 hour. The solid obtained is isolated by filtration and washed with methyl ethyl ketone (10 mL) followed by drying at 60°C under vacuum to afford title compound in 72% yield. HPLC purity is 99.98%.

### EXAMPLE 2: PREPARATION OF RASAGILINE HEMITARTRATE.

Rasagiline free base (7.0 g) and methanol (49 mL) were charged in a flask. To it, a solution of L-(+)-tartaric acid (2.7 g) in methanol (56 mL) was added followed by stirring of mixture for 30 minutes. To this mixture, methyl ethyl ketone (35 mL) was added and mixture was stirred for 10 minutes. The reaction mixture was heated to reflux temperature at 60-65°C and maintained at same for about 60-90 minutes followed by cooling to room temperature and again stirring at same for 60-90 minutes. The solid obtained is isolated by filtration and washed with methyl ethyl ketone (14 mL) followed by drying at 60°C under vacuum to afford title compound in 72% yield. HPLC purity is 99.93%. Specific surface area = 2.36 m²/g

### EXAMPLE 3: PREPARATION OF RASAGILINE HEMITARTRATE (COMPARATIVE EXAMPLE)

Rasagiline hemitartrate (18.5 g), methanol (250 mL) and isopropyl alcohol (250 mL) were charged in a flask. The reaction mixture is heated to reflux followed by addition of 1:1 mixture of methanol and isopropyl alcohol (280 mL) at the same temperature for complete dissolution. The reaction mixture was cooled to about 17°C and solid is isolated by filtration. The solid obtained is washed with 1:1 mixture of methanol and isopropyl alcohol (40 mL) and dried under vacuum at 60-70oC to afford the title compound in ~80% yield. HPLC purity is 99.91%. Specific surface area = 3.94 m²/g

### EXAMPLE 4: PREPARATION OF RASAGILINE HEMITARTRATE

Rasagiline free base (7.0 g) and methanol (49 mL) were charged in a flask. To it, a solution of L-(+)-tartaric acid (2.7 g) in methanol (56 mL) was added followed by stirring of mixture for 30 minutes. To this mixture, isopropyl alcohol (35 mL) was added and mixture was stirred for 10 minutes. The reaction mixture was heated to reflux temperature at 60-65°C and maintained at same for about 60-90 minutes followed by cooling to room temperature and again stirring at same for 60-90 minutes. The solid obtained is isolated by filtration and washed with isopropyl alcohol (14 mL) followed by drying at 60°C under vacuum to afford title compound in 75% yield. HPLC purity is 99.91 %.

### EXAMPLE 5: PREPARATION OF RASAGILINE HEMITARTRATE.

Rasagiline hemitartrate (0.75 kg) and water (15 kg) were charged in a reactor and stirred for 20-30 minutes at 25-35°C. Then the pH of the mixture was adjusted to about 11-12.5 with aqueous sodium hydroxide solution (230 g in 15 kg of water). The mixture was again maintained at 25-35°C for 20-30 minutes followed by addition of ethyl acetate (5.2 L). The mixture was stirred and organic layer was separated. The aqueous layer was extracted with ethyl acetate (5.3 L), the total organic layers were combined and washed twice with water (5.2 L and 5.3 L). The organic layer was separated and subjected to distillation under vacuum at below 55°C to afford the residue. The residue was taken up in methanol (3.2 L), mixture was stirred and filtered through millipore filter under vacuum In a separate flask L-tartaric acid (229 g) was taken in 3.2 L of methanol and subjected to filtration under vacuum through millipore filter. This L-tartaric acid solution was added to above solution over a period of 15-30 minutes. Then methyl ethyl ketone (4.2 L) was added and the mixture was stirred at 25-35°C for 15-30 minutes. The mixture was heated to 60-65oC for about 60-90 minutes followed by cooling of mixture to 25-35oC. The mixture was further maintained at same temperature for 90-120 minutes followed by filtration of solid under vacuum. The solid was washed with methyl ethyl ketone (2.1 L) and suck dried for 30-60 minutes under vacuum followed by drying at 65-70°C under vacuum for about 10 hours to afford Rasagiline hemitartrate in about 70% yield. Aspect Ratio = 23.

## Claims

1. A process for preparation of Rasagiline hemitartrate comprising,
(a) providing a mixture of rasagiline in methanol;
(b) treating the mixture of step a) with L-tartaric acid;
(c) combining the mixture of step (b) with methyl ethyl ketone and maintaining the mixture at a suitable temperature for a suitable time for crystallization;
(d) recovering the solid rasagiline hemitartrate from step (c).

2. The process of claim 1 wherein in step b) L-tartaric acid added is in the form of solid.

3. The process of claim 1 wherein in step b) L-tartaric acid added is in the form of solution in methanol.

4. The process of anyone of claims 1 to 3 wherein in step c) the mixture of step b) is added to methyl ethyl ketone.

5. The process of anyone of claims 1 to 3 wherein in step c) methyl ethyl ketone is added to the mixture of step b).

6. The process of anyone of claims 1 to 5 wherein in step d) the recovery is materialized by filtration.

7. A process for preparation of Rasagiline hemitartrate comprising,
(a) providing a mixture of rasagiline hemitartrate in methanol;
(b) combining the mixture of step (a) with methyl ethyl ketone and maintaining the mixture at a suitable temperature for a suitable time for crystallization;
(c) recovering the solid rasagiline hemitartrate from step (b).

8. The process of claim 7 wherein in step b) the mixture of step a) is added to methyl ethyl ketone.

9. The process of claim 7 wherein in step b) methyl ethyl ketone is added to the mixture of step a).

10. Rasagiline hemitartrate having aspect ratio of more than 15 prepared by process of any of the claims 1-9.
